# EUROPÄISCHE PATENTANMELDUNG

(11) **EP 1 325 710 A2**
(43) Veröffentlichungstag der Anmeldung: **09.07.2003**
(21) Anmeldenummer: 02405959.4
(22) Anmeldetag: 07.11.2002
(51) Int. Cl.: A61B 17/32

(54) **Microchirurgische Schere**

(30) Priorität: 21.12.2001 US 32306
(71) Anmelder: Alcon Grieshaber AG, 8203 Schaffhausen (CH)
(72) Erfinder: Jud, Oliver, 8224 Löhningen (CH); Maag, Werner, 8750 Glarus (CH)
(74) Vertreter: Althoff, Gerhard

(57) **Zusammenfassung**

Es wird ein Schneidinstrument (20) in Form einer Schere vorgeschlagen, welches im wesentlichen ein als Handgriff ausgebildetes Gehäuse (25) mit daran angeordneter Funktionseinheit (35), eine darin gelagerte und als Hohlnadel ausgebildete Sonde (46), eine koaxial darin gelagerte Stange (47) sowie eine am distalen Ende derselben angeordnete Schneidevorrichtung (50) umfasst.

Die Schneidevorrichtung (50) hat zwei in axialer Richtung aus der Sonde (46) herausragende Scherenglieder mit daran angeordneten und entweder bogenförmig oder geradlinig ausgebildeten Schneidblättern (56,61), bei welchen daran angeordnete Schneidkanten in geöffneter Scherenstellung unter spitzem Winkel zueinander angeordnet und infolge einer relativ zu den Scherengliedern in Richtung einer Scherenspitze orientierten Bewegung der Sonde (46) die einander zugewandten Schneidkanten miteinander in Eingriff bringbar sind.

## Beschreibung

Die Erfindung betrifft ein mikrochirurgisches Schneidinstrument in Form einer Schere, welches ein als Handgriff ausgebildetes Gehäuse, eine darin angeordnete Schiebevorrichtung für eine in axialer Richtung orientierte Bewegung, eine als Hohlnadel ausgebildete Sonde mit in axialer Richtung darin gelagerter Stange sowie eine am distalen Ende derselben angeordnete Schneidevorrichtung umfasst.

Die vorliegende Erfindung befasst sich allgemein mit dem Problem chirurgischer Eingriffe in Hohlräumen eines Lebewesens, beispielsweise im Auge eines Lebewesens zur Behandlung retinaler Erkrankungen, welche vielfach bei Hypertonie oder bei vaskulären Veränderungen auftreten. Bei derartigen Erkrankungen können im Bereich sich kreuzender und von einer Haut umhüllter Arterien und Venen sogenannte Venenastverschlüsse auftreten, infolge welcher jeweils die Vene von der daraufliegenden Arterie zusammengedrückt beziehungsweise abgeklemmt wird. Durch Auftrennen (Aufschneiden) sowie Entfernen der Hauthülle können derartige Venenastverschlüsse weitgehend eliminiert beziehungsweise verhindert werden.

Die Durchführung derartiger mikrochirurgischer Eingriffe erfordert eine präzise Funktion des jeweils verwendeten Instruments sowie eine sichere Handhabung desselben. Hierbei ist insbesondere zu berücksichtigen, dass das Instrument exakt der krankhaften Stelle zuführbar und für längere Zeit von der Hand des Chirurgen gehalten und gleichzeitig die momentane Schneideposition gut visuell erkennbar ist.

Bei den allgemein bekannten Schneidinstrumenten kann aufgrund der Formgebung der Scherenblätter das Gewebe beschädigt beziehungsweise vor dem eigentlichen Schnitt verformt und verdrängt werden, so dass die bekannten Schneidinstrumente nicht mehr den Anforderungen der modernen Mikrochirurgie entsprechen und für Operationen feiner Gewebestrukturen, insbesondere für die Netzhautchirurgie nicht geeignet sind.

Der Erfindung liegt die Aufgabe zugrunde, ein Schneidinstrument gemäss der im Oberbegriff des Anspruchs 1 genannten Gattung dahingehend zu verbessern, dass die chirurgischen Eingriffe für den Chirurgen auch bei relativ feinen Strukturen , insbesondere aber bei feinen Netzhautstrukturen erleichtert werden und auf engstem Raum bei maximaler Schonung des Gewebes mit exakten Schnitten durchführbar sind.

Das erfindungsgemässe Schneidinstrument ist gekennzeichnet durch zwei in axialer Richtung orientierte und an dem aus der röhrchenförmigen Sonde herausragenden distalen Ende jeweils mit einem Schneidblatt versehene und entweder bogenförmig abgekröpft oder geradlinig ausgebildete Scherenglieder, bei welchen die an den Schneidblättern vorgesehenen Schneidkanten in geöffneter Scherenstellung über eine von der jeweiligen Blattspitze in Richtung der Sonde orientierte Distanz unter spitzem Winkel zueinander angeordnet und infolge einer relativ zu den Scherengliedern in Richtung einer Scherenspitze orientierten Bewegung der Sonde die einander zugewandten Schneidkanten miteinander in Eingriff bringbar sind.

Mit der Erfindung wird ein Schneidinstrument geschaffen, mittels welchem präzise mikrochirurgische Eingriffe durchführbar sind ohne dabei das Gewebe zu beschädigen. Bei Operationen der Netzhautchirurgie, beispielsweise bei subretinalen Operationen ist die Verwendung des erfindungsgemässen Schneidinstruments von besonderem Vorteil.

Eine weitere Ausgestaltung des erfindungsgemässen Schneidinstruments ist gekennzeichnet durch mindestens einen in axialer Richtung in der röhrchenförmigen Sonde angeordneten Lichtleiter, welcher mit dem einen Ende an eine Lichtquelle angeschlossen und an dem anderen Ende zum Beleuchten des aus der Sonde ragenden distalen Endes der beiden in geöffneter und/oder geschlossener Scherenstellung zueinander angeordneten Schneidblätter ausgebildet ist. Hiermit wird erreicht, dass beim Einführen der Schneidevorrichtung sowie während des chirurgischen Eingriffs mindestens die Scherenspitze beziehungsweise die Blattspitzen der beiden Scherenblätter gezielt beleuchtet werden können und infolge davon das Gewebe sowie der momentane Operationsbereich für den Chirurgen gut sichtbar werden.

Zur optimalen Handhabung des erfindungsgemässen Schneidinstruments ist dem in der Sonde angeordneten Lichtleiter ein mit einem optischen Leiter versehener Optikkanal zugeordnet, mittels welchem während des Einführens sowie des chirurgischen Eingriffs das Bild des momentanen Operations- und Beobachtungsbereichs auf einen Monitorbildschirm übertragbar ist. Diese Ausgestaltung gewährleistet, dass auch bei Eingriffen in kleinen Hohlorganen der Operationsbereich für den Chirurgen visuell gut erkennbar und somit ein exakter Eingriff gewährleistet ist.

An dieser Stelle wird darauf hingewiesen, dass das erfindungsgemässe Schneidinstrument nicht auf das ophthalmologische Anwendungsgebiet beschränkt ist und die Verwendung desselben ohne jegliche Abänderungen für manigfaltige chirurgische Eingriffe in Hohlräumen eines Lebewesens im Rahmen der Erfindung liegen. Ohne den Grundgedanken der Erfindung zu verlassen, sind weitere zweckmässige Ausgestaltungen des Schneidinstruments sowie der einzelnen Funktionselemente und deren Anordnung ebenfalls möglich.

Ausführungsbeispiele der Erfindung sowie zweckmässige Ausgestaltungen derselben ergeben sich aus der folgenden Beschreibung in Verbindung mit der Zeichnung. Es zeigt:
- **Fig.1**: ein schematisch und in grösserem Massstab dargestelltes Auge mit einer in den Glaskörperhohlraum eingeführten Sonde eines Schneidinstruments;
- **Fig.2**: das etwa in räumlicher Ansicht dargestellte Schneidinstrument mit daran angeordnetem Funktionsträger für eine mit zwei Scherengliedern versehene Schneidevorrichtung;
- **Fig.3**: den im Schnitt sowie in grösserem Massstab dargestellten Funktionsträger mit den an einer länglichen Stange angeordneten und in geöffneter Stellung dargestellten Scherengliedern;
- **Fig.4**: den Funktionsträger gemäss Fig.3 mit den an der Stange angeordneten und in geschlossener Stellung dargestellten Scherengliedern;
- **Fig.5**: ein weiteres Ausführungsbeispiel des teilweise dargestellten Funktionsträgers mit der in der röhrchenförmigen Sonde angeordneten Stange und zugeordnetem Lichtleiter und/oder zugeordnetem Optikkanal;
- **Fig.6A**: eine erste Variante der gemäss der in Fig.5 eingezeichneten Linie I-I im Schnitt dargestellten Sonde mit der darin angeordneten Stange und den zugeordneten Lichtleiter;
- **Fig.6B**: eine zweite Variante der gemäss der in Fig.5 eingezeichneten Linie I-I im Schnitt dargestellten Sonde mit der darin angeordneten Stange sowie den zugeordneten Lichtleiter und Optikkanal;
- **Fig.7A**: die gemäss der in Fig.5 eingezeichneten Linie II-II im Schnitt dargestellte erste Variante der Sonde mit der Stange und den zugeordneten Lichtleiter;
- **Fig.7B**: die gemäss der in Fig.5 eingezeichneten Linie II-II im Schnitt dargestellte zweite Variante der Sonde mit der Stange sowie den zugeordneten Lichtleiter und Optikkanal;
- **Fig.8**: das gemäss in Fig.5 eingezeichneter Pfeilrichtung III in Draufsicht dargestellte vordere Teilstück der Schneidevorrichtung mit dem zugeordneten Lichtleiter;
- **Fig.9A**: eine erste Variante der etwa in Ansicht dargestellten Schneidevorrichtung mit den beiden etwa in geöffneter Stellung dargestellten Scherengliedern;
- **Fig.9B**: die in Draufsicht dargestellte Schneidevorrichtung gemäss Fig.9A mit den beiden relativ zueinander gespreizten Scherengliedern;
- **Fig.9C**: ein Teilstück der Schneidevorrichtung gemäss Fig.9B mit weitgehend in der Schneideposition dargestellten Scherengliedern;
- **Fig.9D**: die gemäss der in Fig.9A eingezeichneten Linie IV-IV im Schnitt dargestellte und in der Sonde angeordnete Stange für die Schneidevorrichtung;
- **Fig.9E**: die gemäss der in Fig.9C eingezeichneten Linie V-V im Schnitt dargestellte Sonde mit den beiden in geöffneter Schneideposition dargestellten Scherengliedern;
- **Fig.9F**: das in Ansicht und in grösserem Massstab dargestellte distale Ende der Scherenspitze;
- **Fig.10A**: eine zweite Variante der in Ansicht dargestellten Schneidevorrichtung mit den beiden etwa in geöffneter Stellung dargestellten Scherengliedern;
- **Fig.10B**: die in Ansicht dargestellte Schneidevorrichtung gemäss Fig.10A mit den beiden Scherengliedern in geschlossener Stellung;
- **Fig.10C**: die gemäss Fig.10B in Draufsicht dargestellte Schneidevorrichtung;
- **Fig.11A**: eine dritte Variante der in Ansicht dargestellten Schneidevorrichtung;
- **Fig.11B**: die in Draufsicht dargestellte Schneidevorrichtung gemäss Fig.11A mit den beiden in gespreizter Stellung dargestellten Scherengliedern;
- **Fig.12A**: eine vierte Variante der in Ansicht dargestellten Schneidevorrichtung mit den beiden etwa in geöffneter Stellung dargestellten Scherengliedern;
- **Fig.12B**: die in Draufsicht dargestellte Schneidevorrichtung gemäss Fig.12A mit den beiden Scherengliedern;
- **Fig.12C**: die in Ansicht dargestellte Schneidevorrichtung gemäss Fig.12A mit den in geschlossener Stellung dargestellten Scherengliedern;
- **Fig.13A**: eine weitere Variante der in Ansicht dargestellten Schneidevorrichtung mit den beiden etwa in geöffneter Stellung dargestellten Scherengliedern;
- **Fig.13B**: die in Draufsicht dargestellte Schneidevorrichtung gemäss Fig.13A mit den beiden Scherengliedern;
- **Fig.13C**: die in Ansicht dargestellte Schneidevorrichtung gemäss Fig.13A mit in geschlossener Stellung dargestellten Scherengliedern; und
- **Fig.14**: das in Draufsicht sowie in grösserem Massstab dargestellte distale Ende der beiden etwa in der Schneideposition dargestellten Scherenblätter für die einzelnen Schneidevorrichtungen.

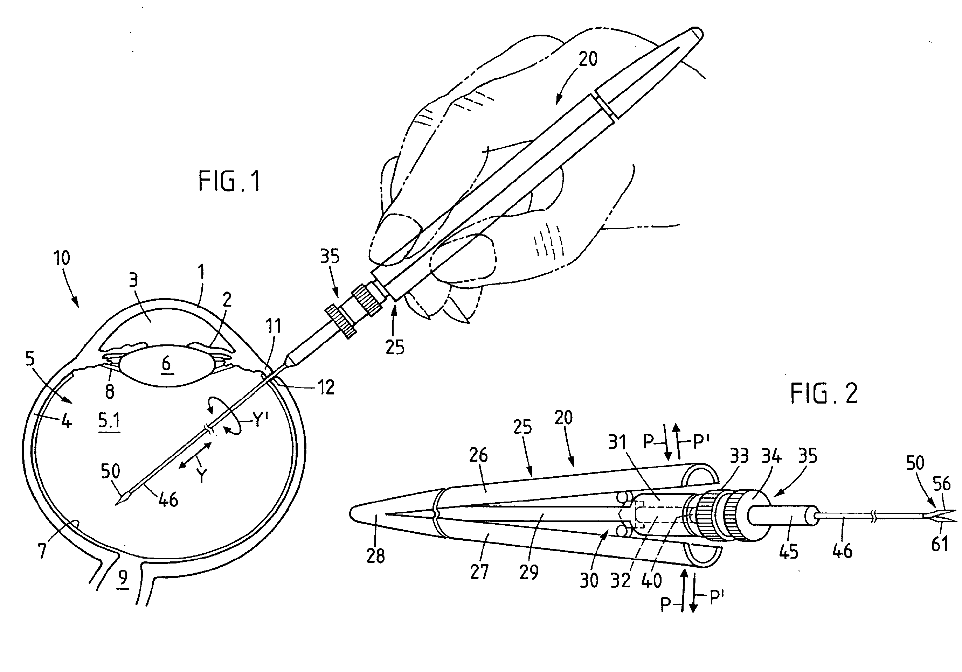

Als Anwendungsbeispiel ist in Fig.1 zur Verdeutlichung der Erfindung ein Auge 10 in grösserem Massstab sowie als Horizontalschnitt dargestellt und man erkennt die Hornhaut 1 (CORNEA), die Regenbogenhaut 2 (IRIS) mit der Pupille 3, die Lederhaut 4 (Sklera), den in der Gesamtheit mit 5 bezeichneten Glaskörper mit dem Glaskörperhohlraum 5.1 und die Linse 6 (LENS), die Netzhaut 7 (RETINA) sowie die Strahlenbänder 8 (ZONULA). Im Bereich des Augenhintergrunds ist das aus dem Auge 10 austretende Sehnervenbündel 9 (OPTICUS) schematisch dargestellt.

Weiterhin erkennt man in Fig.1 ein schematisch dargestelltes Schneidinstrument 20, welches mit einer daran angeordneten Sonde 46 in den Glaskörperhohlraum 5.1 eingeführt ist. Das Schneidinstrument 20 umfasst im wesentlichen ein Gehäuse 25, eine damit wirkverbundene und in Fig.1 nicht näher dargestellte Schiebeeinheit sowie einen Funktionsträger 35 für die als längliche Hohlnadel ausgebildete Sonde 46. Am distalen Ende der Sonde 46 ist eine in Fig.1 schematisch dargestellte Schneidevorrichtung 50 angeordnet.

Für den mikrochirurgischen Eingriff wird von dem Ophthalmologen im Bereich der Pars plana 11 mit einem geeigneten Instrument eine Inzision 12 in die Lederhaut 4 eingeschnitten, durch welche die Sonde 46 mit der Schneidevorrichtung 50 in den Glaskörperhohlraum 5.1 eingeführt werden kann. Nach dem Herausziehen der Sonde 46 zusammen mit der Schneidevorrichtung 50 verschliesst sich die Inzision 12 selbsttätig.

Das Schneidinstrument 20 kann, wie in Fig.1 schematisch dargestellt, mit der Sonde 46 und der Schneidevorrichtung 50 gemäss Doppelpfeilrichtung Y in Bezug auf die zugewandte Innenseite der Netzhaut 7 in axialer Richtung in dem Glaskörperhohlraum 5.1 verschoben sowie um die theoretische Längsachse (nicht dargestellt) gemäss Pfeilrichtung Y' gedreht werden.

Die als längliche Hohlnadel ausgebildete und in den Glaskörperhohlraum 5.1 einführbare Sonde 46 hat einen Aussendurchmesser in der Grössenordnung von etwa 1,0 mm und einen Innendurchmesser von etwa 0,8 mm. In der röhrchenförmigen Sonde 46 ist eine in axialer Richtung orientierte Stange angeordnet, welche an dem aus der Sonde 46 ragenden distalen Ende die zum Schneiden feiner Strukturen ausgebildete Schneidevorrichtung 50 aufweist. Die mit zwei zusammenwirkenden Schneidblättern versehene Schneidevorrichtung 50 sowie zweckmässige weitere Ausgestaltungen derselben wird/werden nachstehend noch im einzelnen beschrieben.

Fig.2 zeigt als bevorzugtes Ausführungsbeispiel das schematisch und etwa in räumlicher Ansicht dargestellte ophthalmologische Schneidinstrument 20 mit der zum Schneiden feiner retinaler Strukturen ausgebildeten Schneidevorrichtung 50. Das Schneidinstrument 20 umfasst ein als Handgriff ausgebildetes und mit zwei halbschalenförmigen, länglichen Gehäuseteilen 26 und 27 versehenes Gehäuse 25. An dem hinteren Ende sind die beiden Gehäuseteile 26 und 27 beispielsweise mittels einer Verschlusskappe 28 in nicht näher dargestellter Weise miteinander verbunden. Zwischen den beiden Gehäuseteilen 26 und 27 ist ein Tragarm 29 sowie eine mit den beiden Gehäuseteilen 26 und 27 zusammenwirkende Schiebevorrichtung 30 angeordnet. Am vorderen Ende des Tragarms 29 ist ein Führungsstück 31 angeordnet, welches zur aufschraubbaren Befestigung des in der Gesamtheit mit 35 bezeichneten Funktionsträgers ausgebildet ist.

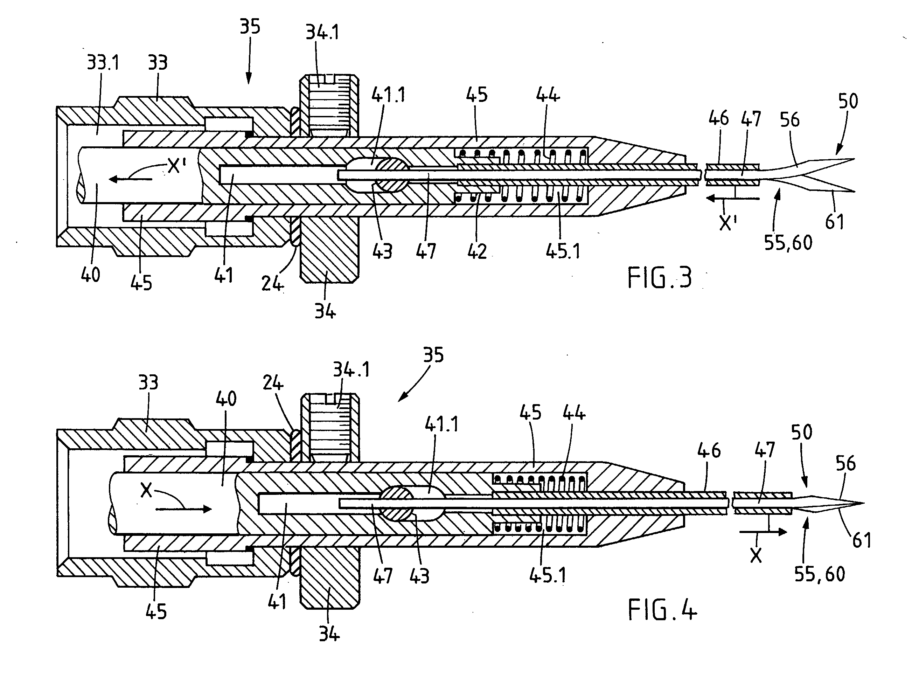

In dem zylindrischen Führungsstück 31 ist ein in Fig.2 schematisch dargestellter und in axialer Richtung orientierter Bolzen 32 gelagert, welcher an dem einen Ende mit der Schiebevorrichtung 30 und an dem anderen Ende mit einem in axialer Richtung orientierten und etwa bolzenförmig ausgebildeten Stellglied 40 wirkverbunden ist. Das Stellglied 40 ist in axialer Richtung verschiebbar in dem Funktionsträger 35 gelagert. Der mit den einzelnen Elementen versehene Funktionsträger 35 ist mittels einer aufschraubbaren Überwurfmutter 33 an dem zylindrischen Führungsstück 31 des Tragarms 29 derart angeordnet und befestigt, dass das bolzenförmige Stellglied 40 mit dem Bolzen 32 der Schiebevorrichtung 30 wirkverbunden ist. Der Funktionsträger 35 wird nachstehend anhand der Figuren 3 und 4 im einzelnen beschrieben.

Bei dem in Fig.2 als Ausführungsbeispiel dargestellten Schneidinstrument 20 wird durch den in Doppelpfeilrichtung P orientierten manuellen Druck auf die beiden Gehäuseteile 26,27 die Schiebevorrichtung 30 betätigt. Hierbei wird der in dem Führungsstück 31 gelagerte Bolzen 32 sowie das damit wirkverbundene Stellglied 40 in axialer Richtung verschoben und infolge davon die mit der Sonde 46 zusammenwirkende Schneidevorrichtung 50 betätigt. Durch Verringerung beziehungsweise Aufhebung des manuellen Drucks auf die beiden Gehäuseteile 26 und 27 werden diese infolge der Rückstellkraft einer in dem Funktionsträger 35 angeordneten und mit dem Stellglied 40 und Bolzen 32 wirkverbundenen Druckfeder gemäss Doppelpfeilrichtung P' zurück bewegt.

Fig.3 zeigt den im Schnitt sowie in grösserem Massstab dargestellten Funktionsträger 35 und man erkennt die mit einer Ausnehmung 33.1 versehene Überwurfmutter 33, eine darin gelagerte Führungshülse 45, einen daran am äusseren Durchmesser angeordneten Zwischenring 24 sowie einen Stellring 34. Der Stellring 34 ist mit einem eingeschraubten Gewindestift 34.1 an der Führungshülse 45 befestigt. In einer zylindrischen Ausnehmung 45.1 der Führungshülse 45 ist das Stellglied 40 angeordnet, welches an dem in der Ausnehmung 45.1 der Führungshülse 45 angeordneten Ende mit einem zylindrischen, abgesetzt ausgebildeten Teilstück 42 versehen ist. An dem zylindrischen Teilstück 42 ist mit dem einen Ende eine zylindrische Druckfeder 44 gelagert, welche mit dem anderen Ende an der Innenwand der Führungshülse 45 abgestützt ist. Die einzelnen Teile 33,24,34,45 und 40 des Funktionsträgers 35 bilden im wesentlichen eine Baueinheit.

Wie weiterhin in Fig.3 dargestellt, ist das Stellglied 40 mit einer in axialer Richtung orientierten Sacklochbohrung 41 sowie einer quer dazu orientierten und mit 41.1 bezeichneten Ausnehmung versehen. Die Sacklochbohrung 41 ist zur Aufnahme und Lagerung einer Stange 47 sowie zur Aufnahme der als Hohlnadel ausgebildeten und am Stellglied 40 befestigten Sonde 46 ausgebildet. Die in axialer Richtung in der röhrchenförmigen Sonde 46 gelagerte Stange 47 ist an dem vorderen distalen Ende mit der Schneidevorrichtung 50 versehen. Die Schneidevorrichtung 50 umfasst im wesentlichen zwei in axialer Richtung orientierte Scherenglieder 55 und 60, welche an dem aus der Sonde 46 ragenden distalen Ende mit etwa scherenförmig zusammenwirkenden Schneidblättern 56 und 61 versehen sind.

Die röhrchenförmige Sonde 46 ist in nicht dargestellter Weise, beispielsweise durch eine Kleb-, Schweiss- oder Lötverbindung mit dem in axialer Richtung verschiebbaren Stellglied 40 wirkverbunden. Die Stange 47 ist an dem in der röhrchenförmigen Sonde 46 gelagerten Ende durch mindestens einen radial in die Führungshülse 45 eingeschraubten Gewindestift 43 gegen axiales Verschieben gesichert. An dem aus der Sonde 46 ragenden Ende ist an der Stange 47 die zum Schneiden feiner Strukturen ausgebildete Schneidevorrichtung 50 angeordnet. In Fig.3 sind die beiden Schneidblätter 56 und 61 infolge der in Pfeilrichtung X' zurückgezogenen Sonde 46 sowie infolge der federelastischen Rückstellkraft der Scherenglieder 55 und 60 in weitgehend geöffneter Stellung dargestellt.

Fig.4 zeigt den vorstehend in Verbindung mit Fig.3 beschriebenen Funktionsträger 35 mit den einzelnen Elementen. Abweichend von Fig.3 ist in Fig.4 das Stellglied 40 entgegen der Rückstellkraft der Druckfeder 44 relativ zu der durch den Gewindestift 43 mit der Führungshülse 45 gesicherten Stange 47 gemäss Pfeilrichtung X in axialer Richtung verschoben. Bei diesem Vorgang werden die beiden Schneidblätter 56 und 61 von der im wesentlichen auf die beiden länglichen Scherenglieder 55 und 60 geschobenen Sonde 46 geschlossen. Die relativ zu der Schneidevorrichtung 50 in axialer Richtung orientierte Bewegung der röhrchenförmigen Sonde 46 wird mittels des in Fig.2 als Ausführungsbeispiel dargestellten und von dem Ophthalmologen beispielsweise manuell betätigten Schneidinstruments 20 erreicht.

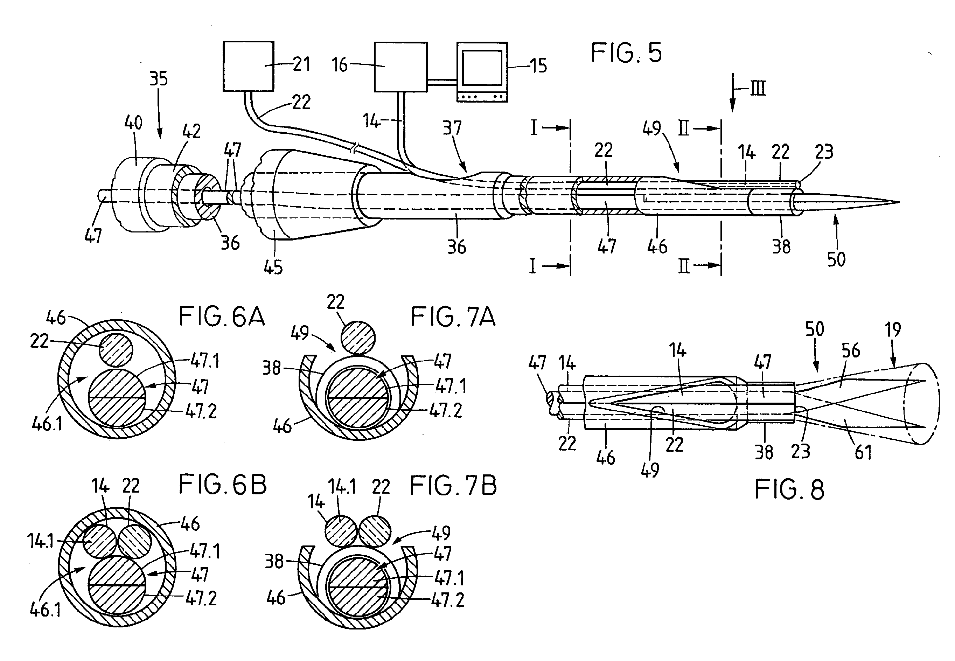

Fig.5 zeigt als weiteres Ausführungsbeispiel ein in Ansicht und teilweise im Schnitt dargestelltes Teilstück des Funktionsträgers 35, welcher weitgehend analog dem vorstehend in Verbindung mit den Figuren 3 und 4 beschriebenen Funktionsträger 35 ausgebildet ist. Abweichend davon ist bei der in Fig.5 dargestellten Variante in der Führungshülse 45 ein erstes Rohrstück 36 gelagert, welches mit dem einen Ende in nicht dargestellter Weise an dem Stellglied 40 befestigt ist. An dem anderen Ende des ersten Rohrstücks 36 ist koaxial die röhrchenförmige Sonde 46 angeordnet und befestigt. An dem vorderen Ende der röhrchenförmigen Sonde 46 ist weiterhin ein zweites Rohrstück 38 exzentrisch angeordnet und befestigt.

Wie weiterhin in Fig.5 dargestellt, ist die am vorderen Ende mit der Schneidevorrichtung 50 versehene und in axialer Richtung orientierte Stange 47 koaxial in dem vorderen zweiten Rohrstück 38 und anschliessend geringfügig exzentrisch in der röhrchenförmigen Sonde 46 sowie dem daran befestigten ersten Rohrstück 36 angeordnet. Mit dem anderen Ende ist die Stange 47, wie in Fig.3 und 4 dargestellt, in der Sacklochbohrung 41 des Stellgliedes 40 angeordnet und durch den eingeschraubten Gewindestift 43 gegen axiales Verschieben gesichert. Das in der Führungshülse 45 gelagerte erste Rohrstück 36 mit der Sonde 46 und dem zweiten Rohrstück 38 bilden zusammen mit dem Stellglied 40 eine in axialer Richtung relativ zu der fixierten Stange 47 und Schneidevorrichtung 50 verschiebbare Baueinheit.

Bei diesem Ausführungsbeispiel ist in dem ersten Rohrstück 36 eine Eintrittsöffnung 37 zum Einführen eines Lichtleiters 22 vorgesehen. Der Lichtleiter 22 wird durch die Eintrittsöffnung 37 in das erste Rohrstück 36 und von dort in die Sonde 46 eingeführt. Der Lichtleiter 22 durchdringt die röhrchenförmige Sonde 46 in axialer Richtung und wird durch eine am Ende derselben vorgesehene Austrittsöffnung 49 herausgeführt. Der aus der Sonde 46 herausgeführte Lichtleiter 22 ist vorzugsweise am äusseren Umfang des zweiten Rohrstücks 38 angeordnet und beispielsweise mit nicht dargestellten Mitteln daran befestigt. Mittels des an der Stirnseite 23 des Lichtleiters 22 austretenden Lichtkegels 19 kann der distale Bereich der beiden Schneidblätter 56 und 61, wie in Fig.8 schematisch dargestellt, beleuchtet werden. Der Lichtleiter 22 steht mit einer in Fig.5 schematisch dargestellten Lichtquelle 21 in Verbindung. Die Lichtquelle 21 kann beispielsweise auch in Form einer Batterie in dem Gehäuse 25 des Instruments 20 (Fig.2) angeordnet werden.

Bei einer weiteren Variante des in Fig.5 dargestellten Ausführungsbeispiels ist in der röhrchenförmigen Sonde 46 beispielsweise ein an sich bekannter und in axialer Richtung orientierter Optikkanal 14 angeordnet. In dem Optikkanal 14 ist zur Bildübertragung, beispielsweise zur monoskopischen Bildübertragung ein optischer Leiter 14.1 angeordnet. Der in dem Optikkanal 14 angeordnete optische Leiter 14.1 steht über eine in die Eintrittsöffnung 37 der Sonde 46 eingeführte Leitung 14 mit einer in Fig.5 schematisch dargestellten Kamera 16 und diese mit einem Monitorbildschirm 15 in Verbindung. Mittels des mit dem distalen Ende auf den Operationsbereich gerichteten optischen Leiters 14.1 können entsprechende Bilder erfasst und von der Kamera 16 zur visuellen Betrachtung des operativen Eingriffs auf den Monitorbildschirm 15 übertragen werden. Der an sich bekannte optische Leiter 14.1 besteht beispielsweise aus einer oder einer Vielzahl zu einem Bündel zusammengefasster Lichtleiterfasern. Bei einer weiteren Variante besteht die Möglichkeit, dass der optische Leiter 14.1 in der Leitung 14 integriert ist und diese in axialer Richtung bis zum distalen Ende in der Sonde 46 angeordnet ist.

Fig.6A zeigt als erste Variante die gemäss der in Fig.5 eingezeichneten Linie I-I im Schnitt sowie in grösserem Massstab dargestellte röhrchenförmige Sonde 46 und man erkennt die im Innenraum 46.1 derselben angeordnete Stange 47 mit den beiden im Profilquerschnitt etwa halbkreisförmig ausgebildeten Armen 47.1 und 47.2 sowie den Lichtleiter 22.

Fig.6B zeigt als zweite Variante die gemäss der in Fig.5 eingezeichneten Linie I-I im Schnitt sowie in grösserem Massstab dargestellte röhrchenförmige Sonde 46 und man erkennt die im Innenraum 46.1 derselben angeordnete und mit den beiden halbkreisförmig ausgebildeten Armen 47.1 und 47.2 versehene Stange 47. Abweichend von der Variante gemäss Fig.6A ist bei dieser Variante zusätzlich zu dem in axialer Richtung der Sonde 46 orientierten Lichtleiter 22 der mit dem optischen Leiter 14.1 versehene Optikkanal 14 angeordnet.

Fig.7A zeigt als erste Variante die gemäss der in Fig.5 eingezeichneten Linie II-II im Schnitt sowie in grösserem Massstab dargestellte Sonde 46 mit der in diesem Bereich angeordneten Austrittsöffnung 49. An der Sonde 46 ist das zweite Rohrstück 38 angeordnet, in welchem die mit den beiden halbkreisförmigen Armen 47,1 und 47.2 versehene Stange 47 koaxial gelagert ist. Weiterhin erkennt man den aus der Ausnehmung 49 herausgeführten und am Aussendurchmesser des zweiten Rohrstücks 38 anliegend angeordneten Lichtleiter 22, welcher vorzugsweise mit nicht dargestellten Mitteln daran befestigt ist.

Fig.7B zeigt als zweite Variante die gemäss der in Fig.5 eingezeichneten Linie II-II im Schnitt sowie in grösserem Massstab dargestellte Sonde 46 mit dem daran angeordneten zweiten Rohrstück 38 sowie die koaxial darin angeordnete Stange 47. Abweichend von der Variante gemäss Fig.7A ist bei dieser Variante zusätzlich zu dem Lichtleiter 22 der mit dem optischen Leiter 14.1 versehene Optikkanal 14 angeordnet. Die aus der Ausnehmung 49 herausgeführten Elemente 22 und 14 sind am Aussendurchmesser des zweiten Rohrstücks 38 anliegend angeordnet und vorzugsweise mit nicht dargestellten Mitteln daran befestigt.

Fig.8 zeigt das gemäss der in Fig.5 eingezeichneten Pfeilrichtung III in Draufsicht sowie in grösserem Massstab dargestellte Teilstück der Sonde 46 mit dem daran angeordneten zweiten Rohrstück 38 und der in axialer Richtung orientierten Austrittsöffnung 49. Weiterhin erkennt man den aus der Austrittsöffnung 49 herausgeführten Lichtleiter 22 mit dem zugeordneten Optikkanal 14, welche beide an dem zweiten Rohrstück 38 anliegend angeordnet sind. Ferner erkennt man in Fig.8 die aus dem zweiten Rohrstück 38 herausgeführte und mit den etwa in geöffneter Stellung dargestellten Schneidblättern 56 und 61 versehene Schneidevorrichtung 50.

Der Lichtleiter 22 ist an der Stirnseite 23 vorzugsweise derart ausgebildet, dass der abgestrahlte Lichtkegel 19 vorzugsweise mit begrenztem Raumwinkel auf das distale Ende der Schneidevorrichtung 50 gerichtet und dadurch die Schneidefunktion während des chirurgischen Eingriffs für den Ophthalmologen gut sichtbar ist.

Bei einem weiteren, bevorzugten Ausführungsbeispiel ist dem Lichtleiter 22 der mit dem optischen Leiter 14.1 versehene Optikkanal 14 zugeordnet, so dass der Operations- und Beobachtungsbereich während des chirurgischen Eingriffs auf den Monitorbildschirm 15 übertragbar und für den Ophthalmologen analog visuell erkennbar ist.

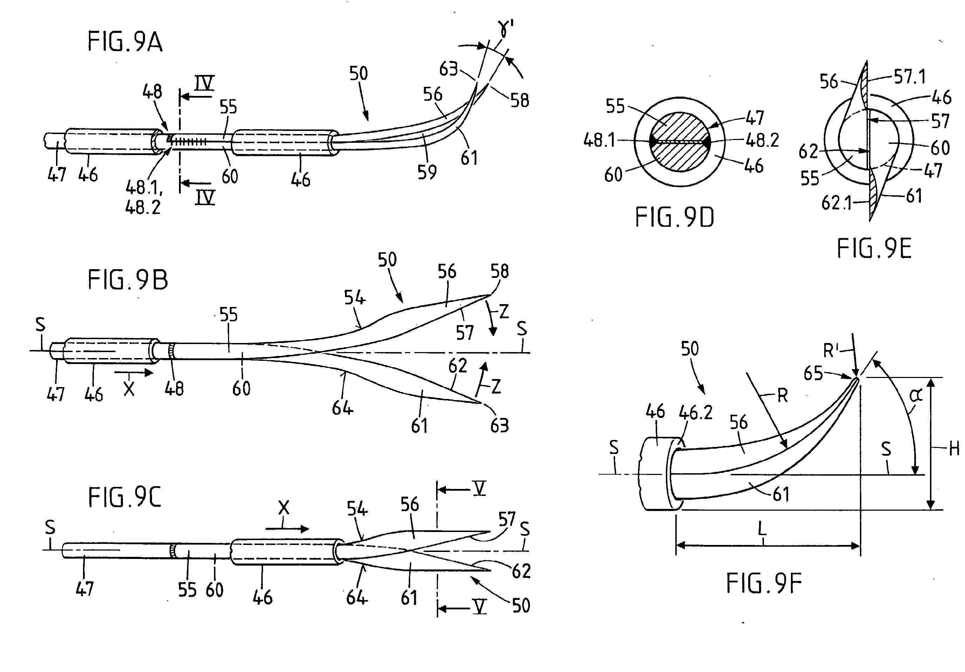

Anhand der Figuren 9A bis 9F wird nachstehend eine erste Variante der Schneidevorrichtung 50 beschrieben. Die in Fig.9A etwa in Ansicht dargestellte Schneidevorrichtung 50 umfasst die in der röhrchenförmigen Sonde 46 angeordnete und im Profilquerschnitt kreisförmig ausgebildete Stange 47 mit den beiden daran angeordneten Scherengliedern 55 und 60. Die beiden länglichen Scherenglieder 55 und 60 sind im vorderen Bereich bogenförmig abgekröpft ausgebildet. Das im Profilquerschnitt halbkreisförmig ausgebildete zweite Scherenglied 60 ist vorzugsweise als Verlängerung an der Stange 47 angeformt. Das ebenfalls im Profilquerschnitt halbkreisförmig ausgebildete erste Scherenglied 55 ist an den Stellen 48,48.1 und 48.2 durch eine Kleb-, Schweiss- oder Lötverbindung an der Stange 47 beziehungsweise an dem zweiten Scherenglied 60 befestigt.

Die beiden gegenüberliegenden Verbindungsstellen 48.1 und 48.2 haben eine in axialer Richtung (Fig.9A) orientierte Länge von etwa 5 mm, so dass die beiden Scherenglieder 55 und 60 im vorderen Bereich entgegen der federelastischen Rückstellkraft gespreizt werden können. Im vorderen Bereich sind die beiden Scherenglieder 55 und 60 jeweils mit dem angeformten länglichen Scherenblatt 56 und 61 versehen. Die beiden mit relativ grossem Radius (Fig.9F) bogenförmig ausgebildeten und zur Erreichung einer Vorspannung relativ zueinander gespreizten Scherenblätter 56 und 61 sind durch einen Spalt 59 beabstandet. In der Stellung gemäss Fig.9A sind die sich im vorderen Bereich kreuzenden Schneidblätter 56,61 mit den beiden daran angeformten Spitzen 58,63 unter einem Verschränkungswinkel γ' in der Grössenordnung von 1° bis 3° zueinander angeordnet, wobei bei einem bevorzugten Ausführungsbeispiel der Verschränkungswinkel γ' = 1,4° beträgt.

In Fig.9B ist die Schneidevorrichtung 50 in Draufsicht dargestellt und man erkennt die in der röhrchenförmigen Sonde 46 angeordnete Stange 47 mit den beiden Scherengliedern 55 und 60 sowie die daran angeformten Scherenblätter 56 und 61. In dieser Position sind die beiden Scherenblätter 56 und 61 mit den daran vorgesehenen Schneidkanten 57,62 und Scherenspitzen 58,63 relativ zu der theoretischen Längsachse S-S gespreizt und im Abstand zueinander angeordnet. Die äusseren Längsseiten 54 und 64 der beiden Scherenglieder 55 und 60 sind derart bogenförmig ausgebildet, dass bei der gemäss Pfeilrichtung X in axialer Richtung orientierten Bewegung der Sonde 46 die beiden Scherenblätter 56 und 61 entgegen der federelastischen Rückstellkraft gemäss Pfeilrichtung Z synchron in Richtung der Längsachse S-S bewegt werden.

Fig.9C zeigt die Schneidevorrichtung 50 gemäss Fig.9B, bei welcher infolge der in axialer Richtung gemäss Pfeilrichtung X verschobenen Sonde 46 die beiden Scherenglieder 55 und 60 mit den angeformten Scherenblättern 56 und 61 teilweise geschlossen sowie die daran angeordneten Schneidkanten 57,62 miteinander in Eingriff gebracht sind.

In Fig.9D sind die gemäss der in Fig.9A eingezeichneten Linie IV-IV im Profilquerschnitt halbkreisförmig ausgebildeten Scherenglieder 55 und 60 der Stange 47 sowie die röhrchenförmige Sonde 46 dargestellt. Die mit den Flachseiten einander zugewandten Scherenglieder 55 und 60 sind, wie vorstehend in Verbindung mit Fig.9A beschrieben, an der Stelle 48 (Fig.9A) sowie an den gegenüberliegenden Stellen 48.1 und 48.2 miteinander verbunden.

Fig.9E zeigt einen Schnitt gemäss der in Fig.9C eingezeichneten Linie V-V und man erkennt die in der Sonde 46 angeordnete Stange 47 und die beiden in geöffneter Stellung dargestellten Scherenglieder 55,60 sowie die beiden Scherenblätter 56 und 61 mit den daran angeordneten und einander zugewandten sowie mit den Schneidkanten 57 und 62 versehenen Schneidflächen 57.1 und 62.1.

In Fig.9F ist in grösserem Massstab das distale Ende der Schneidevorrichtung 50 gemäss der ersten Variante dargestellt und man erkennt die beiden in geschlossener Stellung dargestellten und in Richtung einer abgerundeten Scherenspitze 65 verjüngend ausgebildeten Scherenblätter 56 und 61. Die Scherenspitze 65 ist vorzugsweise mit einem Radius R' in der Grössenordnung von 0,01 mm bis 0,03 mm abgerundet und kann zum Einstechen benutzt werden. Die beiden Scherenblätter 56 und 61 sind als längliche und in axialer Richtung gestreckte Schneidevorrichtung 50 ausgebildet, bei welcher die beiden Scherenblätter 56 und 61 unter spitzem Kröpfungswinkel α und einem Radius R relativ zu der theoretischen Längsachse S-S gleichsinnig gebogen beziehungsweise abgekröpft ausgebildet sind.

Bei der in Fig.9F geschlossen dargestellten Schneidevorrichtung 50 haben die beiden Scherenblätter 56 und 61 ausgehend von der Stirnseite 46.2 der Sonde 46 bis zur Scherenspitze 65 eine Länge **L**, welche grösser als die vom Aussendurchmesser der Sonde 46 bis zur Scherenspitze 65 reichende Höhe **H** ist. Das aus der Sonde 46 ragende Teilstück der Schneidevorrichtung 50 ist gemäss der ersten Variante relativ lang gestreckt und im Abstand zu der Stirnseite 46.2 der Sonde 46 mit dem Kröpfungswinkel α in bezug auf die theoretische Längsachse S-S etwa bogenförmig ausgebildet. Bei dem dargestellten Ausführungsbeispiel beträgt die Länge **L** etwa 2,5 mm bis 3,8 mm und die Höhe **H** etwa 1,7 mm bis 2,0 mm. Der Radius **R** beträgt etwa 1,8 mm bis 2,0 mm und der Kröpfungswinkel α liegt etwa zwischen 40° und 65°.

An dieser Stelle wird darauf hingewiesen, dass bei einem bevorzugten Ausführungsbeispiel die Länge **L**=3,0 mm, die Höhe **H**=1,55 mm, der Radius **R**=2,0 mm und der Kröpfungswinkel α=60° beträgt.

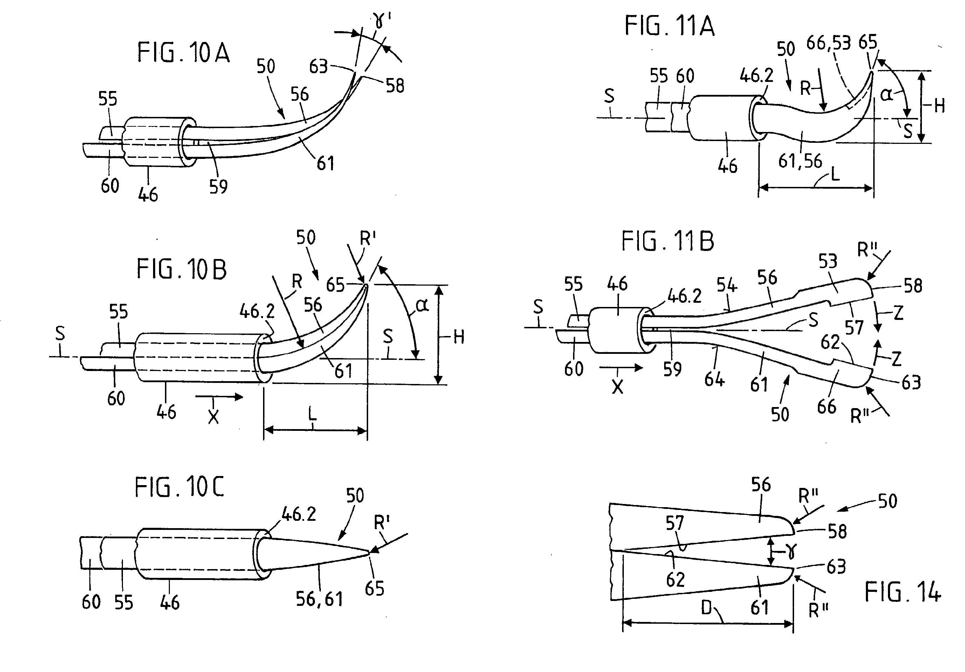

In den Figuren 10A bis 10C ist eine zweite Variante der Schneidevorrichtung 50 dargestellt, welche im wesentlichen analog der ersten Variante ausgebildet ist und die röhrchenförmige Sonde 46, die beiden darin angeordneten Scherenglieder 55 und 60 mit den daran angeformten Scherenblättern 56 und 61 umfasst. Bei der in Fig.10A dargestellten Position der Sonde 46 ist infolge der federelastischen Rückstellkraft zwischen den beiden Scherengliedern 55 und 60 der Spalt 59 vorgesehen und die Blattspitzen 58 und 63 sind etwa in geöffneter Stellung zueinander angeordnet. Durch eine in axialer Richtung orientierte Schiebebewegung der röhrchenförmigen Sonde 46 relativ zu den beiden Scherengliedern 55 und 60 werden die Schneidblätter 56 und 61 entgegen der federelastischen Rückstellkraft für den Schneidevorgang entsprechend geschlossen. In der Stellung gemäss Fig.10A sind die sich im vorderen Bereich kreuzenden Schneidblätter 56,61 mit den beiden daran angeformten Spitzen 58,63 unter einem Verschränkungswinkel γ' in der Grössenordnung von 1° bis 3° zueinander angeordnet, wobei bei einem bevorzugten Ausführungsbeispiel der Verschränkungswinkel γ' = 1,4° beträgt.

Fig.10B zeigt die mit der Sonde 46 und den beiden darin angeordneten Scherengliedern 55,60 versehene Schneidevorrichtung 50 gemäss Fig.10A, bei welcher infolge der gemäss Pfeilrichtung X in axialer orientierten Schiebebewegung der röhrchenförmigen Sonde 46 die beiden in Richtung der abgerundeten Scherenspitze 65 verjüngend ausgebildeten Scherenblätter 56 und 61 geschlossen sind.

Abweichend von der ersten Variante gemäss Fig.9F ist bei der zweiten Variante gemäss Fig.10B die von der Stirnseite 46.2 der Sonde 46 bis zur Scherenspitze 65 reichende gestreckte Länge **L** in Bezug auf die vom Aussendurchmesser der Sonde 46 bis zur Scherenspitze 65 reichende Höhe **H** etwa gleich gross ausgebildet. Der Radius **R** ist vorzugsweise kleiner als die Länge **L** ausgebildet. Das aus der Sonde 46 ragende Teilstück der Schneidevorrichtung 50 ist kürzer ausgebildet und in Bezug auf die theoretische Längsachse S-S unmittelbar an der Stirnseite 46.2 der Sonde 46 mit dem Radius **R** abgekröpft. Bei der zweiten Variante gemäss Fig.10B beträgt die Länge **L** und die Höhe **H** etwa 1,7 mm bis 2,0 mm. Der Radius **R** der beiden gebogenen Scherenglieder 56 und 61 beträgt etwa 1,5 mm bis 1,8 mm, welche Scherenglieder 56,61 in bezug auf die Längsachse S-S in Richtung der Scherenspitze 65 unter einem Winkel α in der Grössenordnung etwa 60° bis 70° vorzugsweise unter einem Winkel α von 65° gleichsinnig und bogenförmig abgekröpft sind.

In Fig.10C ist die zweite Variante der Schneidevorrichtung 50 in Draufsicht dargestellt und man erkennt die röhrchenförmige Sonde 46 mit den beiden Scherengliedern 55,60 sowie die stirnseitig herausragenden und übereinanderliegenden Scherenblätter 56 und 61. Die Scherenblätter 56 und 61 sind ausgehend von der Stirnseite 46.2 der Sonde 46 in Richtung der mit dem Radius R' von etwa 0,01 mm bis 0.03 mm abgerundeten Scherenspitze 65 konisch verjüngend ausgebildet.

In Figur 11A ist eine dritte Variante der Schneidevorrichtung 50 dargestellt und man erkennt die röhrchenförmige Sonde 46 mit den beiden darin angeordneten Scherengliedern 55 und 60. Bei dieser Variante sind die beiden stirnseitig aus der Sonde 46 herausragenden Scherenblätter 56 und 61 etwa hakenförmig ausgebildet und mit einem Radius **R** in Bezug auf die theoretische Längsachse S-S abgekröpft sowie in Richtung der Scherenspitze 65 verjüngend ausgebildet. Bei der dritten Variante beträgt die von der Stirnkante 46' bis zur Scherenspitze 65 reichende, gestreckte die Länge **L** sowie die Höhe **H** etwa 1,5 mm bis 1,8 mm. Der Radius **R** beträgt etwa 1,5 mm und die Scherenspitze 65 ist in Bezug auf die Längsachse S-S unter einem Winkel α in der Grössenordnung von 65° bogenförmig abgekröpft.

Fig.11B zeigt die in Draufsicht dargestellte dritte Variante der Schneidevorrichtung 50 bei welcher die Sonde 46 relativ zu den beiden Scherenblätter 56 und 61 zurückgezogen ist. Infolge der federelastischen Rückstellkraft sind die beiden Scherenblätter 56 und 61 relativ zu der Längsachse S-S gespreizt zueinander angeordnet. Abweichend von der ersten und zweiten Variante ist bei der dritten Variante an jedem distalen Ende der beiden Scherenblätter 56 und 61 ein in Richtung der Längsachse S-S abgesetzt ausgebildetes Teilstück 53 und 66 angeformt. Die beiden Teilstücke 53 und 66 sind an den einander zugewandten Seiten jeweils mit der angeschliffenen Schneidkante 57 beziehungsweise 62 versehen.

Die beiden an den Scherenblättern 56 und 61 angeordneten Teilstücke 53 und 66 sind derart ausgebildet, dass bei der in Pfeilrichtung X relativ zu den Scherengliedern 55,60 orientierten Bewegung der Sonde 46 diese entlang der gegenüberliegenden Längsseiten 54 und 64 der beiden Scherenblätter 56 und 61 gleitet und diese synchron in Richtung der Längsachse S-S gemäss Pfeilrichtung Z derart zusammendrückt, dass die einander zugewandten Schneidkanten 57 und 62 schneidend in Eingriff gebracht werden. Die an den Teilstücken 53 und 66 vorgesehenen Blattspitzen 58 und 63 sind ebenfalls mit dem in der Grössenordnung von 0,01 mm bis 0,03 mm liegenden Radius R" abgerundet.

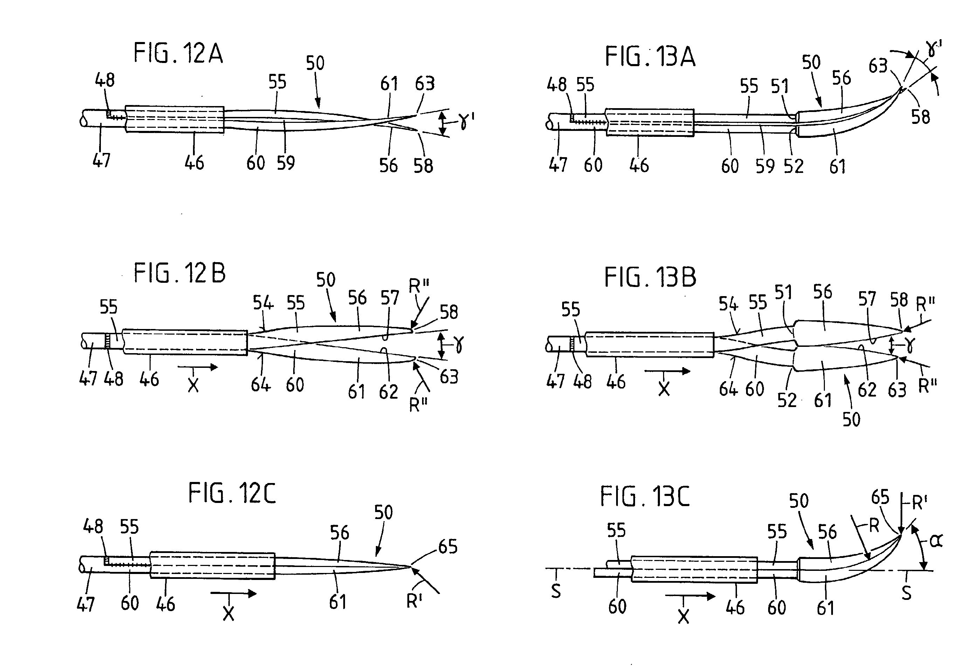

In den Figuren 12A bis 12C ist eine vierte Variante der Schneidevorrichtung 50 in grösserem Massstab dargestellt und man erkennt die röhrchenförmige Sonde 46 sowie die koaxial darin angeordnete Stange 47 mit den beiden Scherengliedern 55 und 60. In Fig.12A sind die beiden durch den Spalt 59 beabstandeten Scherenglieder 55 und 60 mit den daran angeformten Scherenblätter 56,61 und Blattspitzen 58,63 weitgehend in geöffneter Stellung dargestellt. Abweichend von den vorstehend beschriebenen Ausführungsformen gemäss der Figuren 9A und 10A sowie 11A umfasst die Schneidevorrichtung 50 gemäss Fig.12A zwei längliche und geradlinig in axialer Richtung orientierte Scherenglieder 55 und 60 mit den daran angeformten Scherenblättern 56 und 61. In der Stellung gemäss Fig.12A sind die sich im vorderen Bereich kreuzenden Schneidblätter 56,61 mit den beiden daran angeformten Spitzen 58,63 unter einem Verschränkungswinkel γ' in der Grössenordnung von 1° bis 3° zueinander angeordnet, wobei bei einem bevorzugten Ausführungsbeispiel der Verschränkungswinkel γ' = 1,4° beträgt.

Fig.12B zeigt die Schneidevorrichtung 50 gemäss Fig.12A in Draufsicht, bei welcher infolge der in axialer Richtung verschobenen Sonde 46 die beiden mit den Gleit- oder Längsseiten 54,64 versehenen Scherenglieder 55,60 mit den angeformten Scherenblättern 56 und 61 teilweise geschlossen und etwa in der Schneideposition dargestellt sind. In der Schneideposition sind die beiden Schneidkanten 57 und 62 der Scherenblätter 56,61 ausgehend von den beiden mit dem Radius R' abgerundeten Blattspitzen 58 und 63 unter spitzem Öffnungswinkel γ zueinander angeordnet. Die spezielle Ausgestaltung und Abmessungen der einzelnen Elemente wird nachstehend in Verbindung mit Fig.14 im einzelnen beschrieben.

In Fig.12C ist die vierte Variante der Schneidevorrichtung 50 gemäss Fig.12A in Ansicht dargestellt, bei welcher infolge der gemäss Pfeilrichtung X in axialer Richtung orientierten Schiebebewegung der röhrchenförmigen Sonde 46 die beiden in Richtung der abgerundeten Scherenspitze 65 verjüngend ausgebildeten Scherenblätter 56,61 geschlossen sind.

Die Figuren 13A bis 13C zeigen eine weitere und in grösserem Massstab dargestellte Variante der Schneidevorrichtung 50 und man erkennt die röhrchenförmige Sonde 46 sowie die koaxial darin angeordnete Stange 47 mit den beiden an der Verbindungsstelle 48 miteinander verbundenen Scherengliedern 55,60. In Fig.13A sind die beiden durch den Spalt 59 beabstandeten Scherenglieder 55,60 mit den daran angeformten Scherenblättern 56,61 und den beiden Blattspitzen 58,63 in geöffneter Stellung dargestellt. In der Stellung gemäss Fig.13A sind die sich im vorderen Bereich kreuzenden Schneidblätter 56,61 mit den beiden daran angeformten Spitzen 58,63 unter einem Verschränkungswinkel γ' in der Grössenordnung von 1° bis 3° zueinander angeordnet, wobei bei einem bevorzugten Ausführungsbeispiel der Verschränkungswinkel γ' = 1,4° beträgt.

In Fig.13B ist die Schneidevorrichtung 50 gemäss Fig.13A in Draufsicht dargestellt, bei welcher infolge der gemäss Pfeilrichtung X in axialer Richtung verschobenen Sonde 46 die beiden Scherenglieder 55,60 sowie die daran angeordneten Scherenblätter 56,61 teilweise geschlossen beziehungsweise etwa in der Schneideposition dargestellt sind. Die beiden an den Scherengliedern 55,60 angeordneten Scherenblätter 56,61 sind ausgehend von der Übergangsstelle 51 beziehungsweise von der Übergangsstelle 52 in Richtung der jeweiligen Blattspitze 58 beziehungsweise 63 etwa konisch verjüngend weitgehend lanzenförmig ausgebildet. Die beiden einander zugewandten Schneidkanten 57 und 62 sind ausgehend von der jeweils mit dem Radius **R"** abgerundeten Blattspitze 58 und 63 unter spitzem Öffnungswinkel y zueinander angeordnet.

Fig.13C zeigt die Schneidevorrichtung 50 gemäss Fig.13A in Ansicht, bei welcher infolge der gemäss Pfeilrichtung X orientierten Schiebebewegung der röhrchenförmigen Sonde 46 die beiden in Richtung der abgerundeten Scherenspitze 65 verjüngend ausgebildeten Scherenblätter 56 und 61 geschlossen sind. Die beiden Scherenblätter 56,61 bilden in geschlossener Stellung eine relativ kurze Schneidevorrichtung 50, bei welcher die beiden Scherenblätter 56,61 ebenfalls unter spitzem Kröpfungswinkel α mit dem Radius R relativ zu der theoretischen Längsachse S-S bogenförmig und gleichsinnig abgekröpft ausgebildet sind. Die einzelnen Elemente der Variante gemäss Fig.13A bis 13B haben beispielsweise die vorstehend in Verbindung mit Fig.9F oder in Verbindung mit Fig.10B erwähnten Abmessungen.

An dieser Stelle wird darauf hingewiesen, dass die spezielle Ausgestaltung und Abmessungen der distalen Enden für die beiden Scherenblätter 56 und 61 der vorstehend beschriebenen und generell mit 50 bezeichneten Schneidevorrichtung nachstehend anhand von Fig.14 beschrieben wird. Fig.14 zeigt das in Draufsicht sowie in grösserem Massstab dargestellte distale Ende der beiden etwa in der Schneideposition dargestellten Scherenblätter 56 und 61 für die jeweilige Schneidevorrichtung 50. Die beiden Schneidkanten 57 und 62 sind ausgehend von den beiden mit dem Radius **R"** abgerundeten Blattspitzen 58 und 63 über eine Distanz **D** unter spitzem Öffnungswinkel γ zueinander angeordnet. Die Distanz **D** liegt etwa in der Grössenordnung von 1,0 mm bis etwa 2,0 mm und der Öffnungswinkel γ liegt in der Grössenordnung von etwa 9° bis 16°. Bei einer bevorzugten Ausführung beträgt die Distanz **D** = 1,5 mm und der Öffnungswinkel γ = 12°. Hiermit wird erreicht, dass der Schneidevorgang mit relativ kurzem Hub ausschliesslich im äussersten Winkelbereich der beiden geöffneten Scherenblätter 56 und 61 oder Branchen erfolgt.

Die einzelnen vorstehend in Verbindung mit den einzelnen Figuren beschriebenen und an der Stange 47 angeordneten Varianten der Schneidevorrichtung 50 können austauschbar in die Sonde 46 eingesetzt werden, wobei die Stange 47 mittels der jeweils des in die Führungshülse 45 einschraubbaren Gewindestifts 43 fixiert wird und folglich mit der in Fig.3 und Fig.4 dargestellten Funktionseinheit 35 eine Baueinheit bilden.

## Patentansprüche

1. Mikrochirurgisches Schneidinstrument (20) in Form einer Schere, welches ein als Handgriff ausgebildetes Gehäuse (25), eine darin angeordnete Schiebevorrichtung (30) für eine in axialer Richtung orientierte Bewegung, eine als Hohlnadel ausgebildete Sonde (46) mit in axialer Richtung darin gelagerter Stange (47) sowie eine am distalen Ende derselben angeordnete Schneidevorrichtung (50) umfasst **gekennzeichnet durch** zwei in axialer Richtung orientierte und an dem aus der röhrchenförmigen Sonde (46) herausragenden distalen Ende jeweils mit einem Schneidblatt (56,61) versehene und bogenförmig oder geradlinig ausgebildete Scherenglieder (55,60), bei welchen die an den Schneidblättern (56,61) vorgesehenen Schneidkanten (57,62) in geöffneter Scherenstellung über eine von der jeweiligen Blattspitze (58,63) in Richtung der Sonde (46) orientierte Distanz (D) unter spitzem Winkel (γ) zueinander angeordnet und infolge einer relativ zu den Scherengliedern (55,60) in Richtung einer Scherenspitze (65) orientierten Bewegung der Sonde (46) die einander zugewandten Schneidkanten (57,62) miteinander in Eingriff bringbar sind.

2. Mikrochirurgisches Schneidinstrument nach Anspruch 1, **gekennzeichnet durch** mindestens einen in axialer Richtung in der röhrchenförmigen Sonde (46) angeordneten Lichtleiter (22), welcher mit dem einen Ende an eine Lichtquelle (21) angeschlossen und an dem anderen Ende zum Beleuchten des aus der Sonde (46) ragenden distalen Endes der beiden in geöffneter und/oder geschlossener Scherenstellung zueinander angeordneten Schneidblätter (56,61) ausgebildet ist.

3. Mikrochirurgisches Schneidinstrument nach Anspruch 1, **gekennzeichnet durch** mindestens einen in axialer Richtung in der röhrchenförmigen Sonde (46) angeordneten optischen Leiter (14.1), welcher mit dem einen aus der Sonde (46) ragenden distalen Ende zum optischen Erfassen des momentanen Operationsbereichs und mit dem anderen Ende an eine mit einem Monitorbildschirm (15) zusammenwirkende Kamera (16) zur visuellen Darstellung entsprechend erfasster Bilder angeschlossen ist.

4. Mikrochirurgisches Schneidinstrument nach Anspruch 1, **dadurch gekennzeichnet, dass** mindestens die beiden aus der röhrchenförmigen Sonde (46) ragenden Scherenblätter (56,61) in Bezug auf die theoretische Längsachse (S-S) der Sonde (46) unter spitzem Kröpfungswinkel (α) sowie mit einem Radius (R) bogenförmig und gleichsinnig in Richtung der Scherenspitze (65) gebogen sind.

5. Mikrochirurgisches Schneidinstrument nach Anspruch 4, **dadurch gekennzeichnet, dass** die von der Stirnseite (46.2) der Sonde (46) in Richtung der Längsachse (S-S) orientierte und bis zur Scherenspitze (65) reichende Länge (L) der beiden bogenförmigen Scherenblätter (56,61) gleich gross oder grösser als die vom Aussendurchmesser der Sonde (46) bis zur Scherenspitze (65) reichende Höhe (H) ist.

6. Mikrochirurgisches Schneidinstrument nach Anspruch 4, **dadurch gekennzeichnet, dass** die von der Stirnseite (46.2) der Sonde (46) in Richtung der Längsachse (S-S) orientierte und bis zur Scherenspitze (65) reichende Länge (L) der beiden bogenförmigen Scherenblätter (56,61) kleiner als die vom Aussendurchmesser der Sonde (46) bis zur Scherenspitze (65) reichende Höhe (H) ist.

7. Mikrochirurgisches Schneidinstrument nach Anspruch 1, **dadurch gekennzeichnet, dass** die an den Scherengliedern (55,60) angeordneten Schneidblätter (56,61) in Richtung der Scherenspitze (65) geradlinig ausgebildet und mit den an den Schneidblättern (56,61) angeordneten Schneidkanten (57,62) bei geöffneter Scherenstellung ausgehend von der Scherenspitze (65) in Richtung der Sonde (46) über eine Distanz (D) unter spitzem Winkel (γ) zueinander angeordnet sind.

8. Mikrochirurgisches Schneidinstrument nach Anspruch 1, **dadurch gekennzeichnet, dass** im Abstand von der Stirnseite (46.2) der Sonde (46) an jedem Scherenglied (55,60) ein etwa lanzenförmig und in Richtung der jeweiligen Blattspitze (58,63) konisch verjüngend ausgebildetes Scherenblatt (56,61) angeordnet ist, und dass die beiden Scherenblätter (56,61) in Bezug auf die theoretische Längsachse (S-S) der Sonde (46) unter spitzem Kröpfungswinkel (α) und mit einem Radius (R) bogenförmig und gleichsinnig in Richtung der Scherenspitze (65) gebogen sind.

9. Mikrochirurgisches Schneidinstrument nach Anspruch 8, **dadurch gekennzeichnet, dass** die an den in Richtung der jeweiligen Blattspitze (58,63) konisch verjüngend ausgebildeten Schneidblätter (56,61) angeordneten Schneidkanten (57,62) bei geöffneter Scherenstellung ausgehend von der Scherenspitze (65) in Richtung der Sonde (46) über eine Distanz (D) unter spitzem Winkel (γ) zueinander angeordnet sind.

10. Mikrochirurgisches Schneidinstrument nach Anspruch 1, **gekennzeichnet durch** zwei aus der Sonde (46) herausragende und etwa in Form eines bogenförmigen Hakens ausgebildete Scherenblätter (56,61), welche in Richtung der Scherenspitze (65) verjüngend ausgebildet und an den einander zugewandten Innenseiten jeweils mit einem Teilstück (53,66) und daran angeordneten Schneidkanten (57,62) die bei der in Richtung der Scherenspitze (65) orientierten Bewegung der Sonde (46) miteinander in Eingriff bringbar sind.

11. Mikrochirurgisches Schneidinstrument nach Anspruch 10, **dadurch gekennzeichnet, dass** die von der Stirnseite (46.2) der Sonde (46) in Richtung der Längsachse (S-S) orientierte und bis zur Scherenspitze (65) reichende gestreckte Länge (L) der beiden hakenförmigen Scherenblätter (56,61) in Bezug auf die von der bogenförmigen Aussenkante der Scherenblätter (56,61) bis zur Scherenspitze (65) reichende Höhe (H) gleich gross oder grösser ausgebildet ist.

12. Mikrochirurgisches Schneidinstrument nach Anspruch 1, **dadurch gekennzeichnet, dass** die beiden aus der röhrchenförmigen Sonde (46) ragenden Scherenblätter (56,61) in Bezug auf die theoretische Längsachse (S-S) der Sonde (46) mit dem Kröpfungswinkel (α) von etwa 40° bis 65° und mit dem Radius (R) von etwa 1,8 mm bis 2,0 mm bogenförmig und gleichsinnig in Richtung der Scherenspitze (65) gebogen sind, und dass die von der Stirnseite (46.2) der Sonde (46) bis zur Scherenspitze (65) reichende Länge (L) etwa 2,5 mm bis 3,8 mm und die vom Aussendurchmesser der Sonde (46) bis zur Scherenspitze (65) reichende Höhe (H) etwa 1,7 mm bis 2,0 mm beträgt.

13. Mikrochirurgisches Schneidinstrument nach Anspruch 1, **dadurch gekennzeichnet, dass** die beiden aus der röhrchenförmigen Sonde (46) ragenden Scherenblätter (56,61) in Bezug auf die theoretische Längsachse (S-S) der Sonde (46) mit dem Kröpfungswinkel (α) 60° und mit dem Radius (R) von 2,0 mm bogenförmig und gleichsinnig in Richtung der Scherenspitze (65) gebogen sind, und dass die von der Stirnseite (46.2) der Sonde (46) bis zur Scherenspitze (65) reichende Länge (L) 3,0 mm und die vom Aussendurchmesser der Sonde (46) bis zur Scherenspitze (65) reichende Höhe (H) 1,55 mm beträgt.

14. Mikrochirurgisches Schneidinstrument nach Anspruch 1, **dadurch gekennzeichnet, dass** die beiden aus der röhrchenförmigen Sonde (46) ragenden Scherenblätter (56,61) unmittelbar an der Stirnseite (46.1) in Bezug auf die theoretische Längsachse (S-S) der Sonde (46) mit einem Kröpfungswinkel (α) von etwa 65° und mit einem Radius (R) von etwa 1,5 mm bis 1,8 mm bogenförmig und gleichsinnig in Richtung der Scherenspitze (65) gebogen sind, und dass die von der Stirnseite (46.2) der Sonde (46) bis zur Scherenspitze (65) reichende Länge (L) sowie die vom Aussendurchmesser der Sonde (46) bis zur Scherenspitze (65) reichende Höhe (H) jeweils 1,5 mm bis 1,8 mm beträgt.

15. Mikrochirurgisches Schneidinstrument nach Anspruch 1, **dadurch gekennzeichnet, dass** bei geöffneter Scherenstellung die von der jeweiligen Blattspitze (58,63) der Schneidblätter (56,61) in Richtung der Sonde (46) orientierte Distanz (D) etwa 1,0 mm bis 2,0 mm, vorzugsweise 1,5mm beträgt und der Öffnungswinkel (γ) etwa 9° bis 16°, vorzugsweise 12° beträgt.

16. Mikrochirurgisches Schneidinstrument nach Anspruch 1, **dadurch gekennzeichnet, dass** die beiden in geöffneter Stellung sich kreuzenden Scherenblätter (56,61) mit den jeweils daran angeformtem Spitzen (58,63) unter einem Verschränkungswinkel (γ') in der Grössenordnung von 1° bis 3°, vorzugsweise mit einem Verschrängkungswinkel (γ') von 1,4° zueinander angeordnet sind.

17. Mikrochirurgisches Schneidinstrument nach Anspruch 1, **dadurch gekennzeichnet, dass** die beiden Scherenblätter (56,61) mit den jeweils daran angeformten Spitzen (58,63) in geschlossener Scherenstellung gemeinsam die mit einem Radius (R') von 0,01 mm bis 0,03 mm abgerundete Scherenspitze (65) bilden.

18. Mikrochirurgisches Schneidinstrument nach Anspruch 1, **gekennzeichnet durch** die Kombination der folgenden Merkmale:
**a)** den beiden an der Stange (47) angeordneten Scherengliedern (55,60) ist ein im Abstand dazu die röhrchenförmige Sonde (46) in axialer Richtung durchdringender sowie an dem einen Ende an eine Lichtquelle (21) angeschlossener Lichtleiter (22) zugeordnet;
**b)** der am distalen Ende aus der Sonde (46) herausgeführte Lichtleiter (22) ist zum Beleuchten der beiden in geöffneter und/oder geschlossener Scherenstellung zueinander angeordneten und bogenförmig oder geradlinig geformten Schneidblätter (56,61) ausgebildet;
**c)** achsparallel zu dem Lichtleiter (22) ist ein die röhrchenförmige Sonde (46) in axialer Richtung durchdringender und mit einem optischen Leiter (14.1) versehener Optikkanal (14) angeordnet;
**d)** der aus der Sonde (46) herausgeführte Optikkanal (14) mit dem optischen Leiter (14.1) ist am distalen Ende zum optischen Erfassen des momentanen Operationsbereichs ausgebildet; und
**e)** der Optikkanal (14) mit dem optischen Leiter (14.1) ist mit dem anderen Ende an eine mit einem Monitorbildschirm (15) in Verbindung stehende Kamera (16) zur visuellen Darstellung vom Operationsbereich erfasster Bilder angeschlossen.

19. Mikrochirurgisches Schneidinstrument nach Anspruch 1, **gekennzeichnet durch** die Verwendung als ophthalmologische Schere, insbesondere zum Einführen in den Glaskörperhohlraum (5.1) eines Auges (10) sowie zur Behandlung retinaler Erkrankungen.

20. Mikrochirurgisches Schneidinstrument nach Anspruch 1, **gekennzeichnet durch** die Verwendung als chirurgische Schere zum Einführen und zur Durchführung chirurgischer Eingriffe in Hohlräumen eines Lebewesens.

21. Mikrochirurgisches Schneidinstrument nach Anspruch 17, **gekennzeichnet durch** die Verwendung als ophthalmologische Schere, insbesondere zum Einführen in den Glaskörperhohlraum (5.1) eines Auges (10) sowie zur Behandlung retinaler Erkrankungen.

22. Mikrochirurgisches Schneidinstrument nach Anspruch 17, **gekennzeichnet durch** die Verwendung als chirurgische Schere zum Einführen und zur Durchführung chirurgischer Eingriffe in Hohlräumen eines Lebewesens.
